# EUROPEAN PATENT SPECIFICATION

(11) **EP 1 933 861 B1**
(45) Date of publication and mention of the grant of the patent: **08.08.2012**
(21) Application number: 06794084.1
(22) Date of filing: 22.09.2006
(51) Int. Cl.: A61K 38/21, C07K 14/565, A61P 29/00

(54) **METHOD FOR TREATING OR PREVENTING MULTI-ORGAN FAILURE**
VERFAHREN ZUR BEHANDLUNG ODER PRÄVENTION VON MULTI-ORGAN-VERSAGEN
PROCÉDÉ DE TRAITEMENT OU DE PRÉVENTION DES DÉFAILLANCES POLYVISCÉRALES

(30) Priority: 07.10.2005 FI 20051003
(43) Date of publication of application: 25.06.2008
(73) Proprietor: Faron Pharmaceuticals OY, 20520 Turku (FI)
(72) Inventor: JALKANEN, Sirpa, 20760 Piispanristi (FI)
(74) Representative: Öhman, Ann-Marie
(86) International application number: PCT/FI2006/000308
(87) International publication number: WO 2007/042602

(56) References cited:
- WO-A-02/100429
- WO-A-03/024399
- WO-A-2004/084933
- WO-A1-2004/084933
- WO-A2-02/080953
- WO-A2-02/089828
- WO-A2-03/075944
- US-A1- 2006 034 801
- US-A1- 2006 198 821
- SAVIC V ET AL: "Induction of ecto-5'-nucleotidase of rat cultured mesangial cells by interleukin-1beta and tumour necrosis factor-alpha" IMMUNOLOGY, BLACKWELL PUBLISHING, OXFORD, GB, vol. 70, 1 January 1990 (1990-01-01), pages 321-326, XP002979250 ISSN: 0019-2805
- VELDHUIS W.B. ET AL.: 'Interferon-Beta Blocks Infiltration of Inflammatory Cells and Reduces Infarct Volume After Ischemic Stroke in the Rat' J. CEREB. BLOOD FLOW METAB. vol. 23, no. 9, September 2003, pages 1029 - 1039, XP008125468
- VELDHUIS W.B. ET AL.: 'Interferon-Beta Prevents Cytokine-Induced Neutrophil Infiltration and Attenuates Blood-Brain Barrier Disruption' J. CEREB. BLOOD FLOW METAB. vol. 23, no. 9, September 2003, pages 1060 - 1069, XP008125466
- LIU H. ET AL.: 'Interferon-beta administration confers a beneficial outcome in a rabbit model of thromboembolic cerebral ischemia' NEUROSCI. LETT. vol. 327, no. 2, July 2002, pages 146 - 148, XP002265673
- FERRO J.M. ET AL.: 'Other Neuroprotective Therapies on Trial in Acute Stroke' CEREBROVASC. DIS. vol. 21, no. SUPPL. 2, 2006, pages 127 - 130, XP008125510
- MAIER C.M. ET AL.: 'Interferon-beat Fails to Protect in a Model of Transient Focal Stroke' STROKE vol. 37, no. 4, April 2006, pages 1116 - 1119, XP003012393

## Description

This invention relates to a method for prevention or treatment of multi-organ failure in an individual by administering to said individual an effective amount of an interferon beta without the simultaneous administration of one or more agents affecting the adenosine level in the individual.

### BACKGROUND OF THE INVENTION

Several conditions, including abdominal injuries, bowel infraction, cardiovascular surgery and shock, can lead to intestinal ischemia-reperfusion injury (IRI).
Importantly, besides causing local injury IRI also triggers systemic inflammatory response in remote organs resulting in a syndrome called multi-organ failure. In this syndrome lungs are especially vulnerable. The most prominent signs of the injury are increased vascular permeability (vascular leakiness) and neutrophil accumulation. The lung injury subsequent to intestinal IRI is primarily due to release of pro-inflammatory cytokines in the gut. Intestinal IRI increases intestinal permeability with subsequent release of bacterial endotoxin that promotes systemic inflammation in multi-organ failure. Also other mediators released from activated neutrophils play an important role.

CD73 (ecto-5'-nucleotidase, 5'-NT) is a glycoprotein expressed on the surface of lymphocytes and endothelial and epithelial cells. CD73 regulates leukocyte adhesion via its enzymatic function. It catalyzes hydrolysis of AMP to adenosine. Adenosine produced by CD73 decreases vascular permeability and neutrophil sequestration in hypoxic tissue. However, the role of CD73 in distant organ injury in IRI is not known.

Due to the central role of vascular leakage in IRI, molecules regulating the permeability changes via adenosine or by other mechanisms may be potential targets to combat multi-organ failure. Since interferon beta is known to both induce CD73 expression (and adenosine production) and has other immunomodulatory effects, we studied its utility in distant organ injury in multi-organ failure.

The published international patent application WO 2004/084933 discloses the use of cytokines for inducing endothelial CD73 expression and subsequently elevating the adenosine level in an individual. The use of interferon beta in combination with adenosine monophosphate (AMP) in the treatment of multi-organ failure in rats is described.

The published US patent application US 2004/0105843 concerns the use of interferon beta in hypoxia/ischemia related blood flow resistance in a patient. The method aims at prevention of blood flow resistance such as clogging. However, this document does not mention anything about treatment or prevention of ischemia reperfusion injury. Reperfusion injury refers to tissue damage caused when blood flow returns to the tissue after a period of ischemia. The absence of oxygen and nutrients from blood has created a condition in which the restoration of blood circulation can result in inflammation and oxidative damage from the oxygen rather than restoration of normal funtion.

The inventor has now surprisingly found that multi-organ failure or ischemia reperfusion injury can successfully be prevented or treated by use of plain interferon beta only, i.e. without simultaneous administration of AMP, adenosine diphosphate (ADP) or adenosine triphosphate (ATP) or any other agent affecting the adenosine level in individual.

### SUMMARY OF THE INVENTION

Thus, this invention concerns a method for prevention or treatment of multi-organ failure in an individual by administering to said individual an effective amount of an interferon beta.

### DETAILED DESCRIPTION OF THE INVENTION

### Definitions and preferable embodiments:

The term "treatment" or "treating" shall be understood to include complete curing of a disease as well as amelioration or alleviation of said disease.

The term "interferon beta" shall be understood to include any interferon beta. Thus, it shall cover any subtype thereof, such as interferon beta 1a, interferon beta 1b, etc., and mixtures thereof.

The term "prevention" shall be understood to include complete prevention, prophylaxis, as well as lowering the individual's risk of falling ill with said disease. This term shall also be understood to include preconditioning of tissue by administering an interferon beta according to the method of this invention at a very early stage (e.g. before operations, before complete diagnosis at stroke and infarct patients) so as to prevent the tissue from damages.

The term "individual" refers to a human or animal subject.

The expression "effective amount" is meant to include any amount of an agent according to the present invention that is sufficient to bring about a desired therapeutical result, especially upon administration to an animal or human subject.

Although it may be likely that the therapeutic effect of interferon beta is mediated by an elevation of the adenosine level, optionally due to increased expression of CD73, followed upon administrating of interferon beta, it should be stressed that also other alternative mechanisms could be involved, especially in multi-organ failure. Therefore, the effect of interferon beta shall be understood not to be restricted to any particular mechanism of action.

According to one preferable embodiment, the administration of interferon beta is started as soon as a trauma patient or infarction or stroke patient is brought to care, optionally even if the final diagnosis is not fully clarified. In case of surgical operations it may be useful to start administering of interferon beta already before the operation, for example 12 h before the start of the operation.

### Therapeutically effective amounts, administration routes and dosage forms:

The therapeutically effective amount of the interferon beta according to this invention to be given to a patient in need of such treatment may depend upon a number of factors including, for example, the age and weight of the patient, the precise condition requiring treatment and its severity, and the route of administration. The precise amount will ultimately be at the discretion of the attending physician. Thus, practice of the present invention may involve any dose, combination with other therapeutically effective drugs, pharmaceutical formulation or delivery system for oral, topical, inhalation or parenteral administration.

Amounts and regimens for the administration of the agents according to the present invention can be determined readily by those with ordinary skill in the art of treating inflammation-related disorders, such as reperfusion injuries, stroke, organ transplantation, traumas, or multi-organ failure syndrome.

Based on this invention it can be assumed that one form of action of e.g. subcutaneously, intramuscularly, intravenously or transdermally given interferon beta is to increase local concentration of adenosine, which is anti-inflammatory. This overcomes the problems related to the use of adenosine, which has a very short half-life and is therefore, not optimal for therapeutic use.

The interferon beta may according to the present invention preferably be administered by infusion or by injection. Intravascular infusions are normally carried out using parenteral solutions contained within an infusion bag or bottle, and may be connected to different systems to control the rate of administration of the parenteral solution. The interferon beta may according to the present invention alternatively be administered as an aerosol.

Preferred formulations for infusion or injection may include carriers, such as human serum albumin, pharmaceutically acceptable salts, buffers, such as phosphates and/or other pharmaceutically acceptable excipients. The active ingredient, the interferon beta may be provided in amounts ranging from e.g., 1-50 x 10⁶ IU per ml. The formulation may preferably be provided as lyophilised powder in dosage form, to be prepared by the addition of water or other solutions suitable for injection prior to the administration.

Interferon beta can be given to the patients suffering from or being at risk of getting inflammations. Those types of inflammatory conditions are for example ischemia reperfusion injuries during the stroke and myocardial infarction. Also organ transplantation and trauma are occasions often associated with major inflammatory components.

The invention will be illuminated by the following non-restrictive Example.

### Example 1

### Treatment of mice with plain interferon beta in multi-organ failure

Weight-, sex- and age-matched C57Black mice were used in this study.

### Preoperative treatment and surgical procedure

Mice were treated on three concequtive days before the induction of the multi-organ failure with subcutaneous injections of interferon-beta (6000 IU/dose) or PBS. For the operation, the mice were anesthesized with ketamine hydrochloride (100 mg/kg of body weight, IP) and xylazine (10 mg/kg of body weight, IP). Superior mesenteric artery was dissected via laparotomy and occluded by microvascular clamp for 30 minutes. During the procedure total of 2 ml of sterile saline was subcutaneously injected into the mice to compensate for the fluid loss by evaporation. The microvascular clamp was released after the ischemia period. Animals were sacrificed after 4 hours of reperfusion and tissue samples were collected.

### Analysis of Vascular Leakage in the Lungs

Mice received intravenously fluorescein-conjugated dextran (molecular weight 70 kDa, 25 mg/kg of body weight in 0,2 ml of sterile saline) 5 minutes prior to sacrifice. This fluorescent dye does not leak out from intact vessels. 7 micrometer cryo-sections were cut from lung tissue samples and examined in a fluorescent microscope. Intensity of tissue fluorescence exceeding a pre-set threshold and area of leakiness was calculated from the images collected by digital camera at x 200 magnification using Image J computer software.

### Results:

In control treated mice FITC-dextran was detected outside the vessels as an indication of vascular damage and leakiness of the endothelial cell barrier. In contrast, no leakiness outside the vasculature was seen in mice treated with interferon-beta. Table 1 summarizes the results when counted as an area of leakiness and Table 2 as an intensity of fluorescence above the pre-set threshold value. Thus, interferon beta treatment protects the animals from the adverse effects of multi-organ failure in lungs. These data show that interferon beta treatment is useful as a profylaxis in conditions predisposing to multi-organ failure (surgery, injury) and it may be useful for treatment of multi-organ failure in an already ongoing disease.

**Table 1. Leakage area**

| | | | |
|---|---|---|---|
| IFN-beta treatment | - (n=13) | + (n=8) | |
| Mean±SEM | 9±2.5%^{a)} | 0.0±0.0% | P<0.0001 |

| | | | |
|---|---|---|---|
| ^{a)}% of total area | | | |

**Table 2. Fluorescence intensity**

| | | | |
|---|---|---|---|
| IFN-beta treatment | - (n=13) | + (n=8) | |
| Mean±SEM | 26.77±4.91^{a)} | 2.03±1.27 | P<0.0001 |

| | | | |
|---|---|---|---|
| ^{a)} Mean fluorescence intensity | | | |

It will be appreciated that the methods of the present invention can be incorporated in the form of a variety of embodiments, only a few of which are disclosed herein. It will be apparent for the expert skilled in the field that other embodiments exist. Thus, the described embodiments are illustrative and should not be construed as restrictive.

## Claims

1. The use of a therapeutically active agent for the manufacture of a pharmaceutical preparation for prevention or treatment of multi-organ failure in an individual, wherein said agent is plain interferon beta and wherein said agent is used without simultaneous administration of one or more agent affecting the adenosine level in the individual.
A

## Patentansprüche

1. Verwendung eines therapeutisch aktiven Mittels für die Herstellung eines pharmazeutischen Präparats zur Prävention oder Behandlung von Multiorganversagen bei einem Individuum, wobei das Mittel einfaches Interferon-beta ist und wobei das Mittel ohne gleichzeitige Verabreichung eines Mittels oder mehrerer Mittel, das/die den Adenosinspiegel in dem Individuum beeinträchtigt/beeinträchtigen, verwendet wird.

## Revendications

1. Utilisation d'un agent thérapeutiquement actif pour la fabrication d'une préparation pharmaceutique pour la prévention ou le traitement de défaillance multiviscérale chez un individu, **caractérisé en ce que** ledit agent est le bêta interféron seulement et ledit agent est utilisé sans administration simultanée d'un ou plusieurs agents affectant le niveau d'adénosine chez l'individu.
